(19) Europäisches Patentamt European Patent Office Office européen des brevets

(11) **EP 1 859 731 B1**

(12) **EUROPEAN PATENT SPECIFICATION**

(45) Date of publication and mention
of the grant of the patent:
**12.08.2009 Bulletin 2009/33**

(51) Int Cl.:
***A61B 5/00*** (2006.01)

(21) Application number: **07010287.6**

(22) Date of filing: **23.05.2007**

(54) **Biological response prediction system, method for predicting biological response and computer program product**

System zur Vorhersage biologischer Reaktionen, Verfahren zur Vorhersage biologischer Reaktionen und Computerprogramm

Système de prédiction de réponse biologique, procédé de détection de réponse biologique et produit de programme informatique

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **24.05.2006 JP 2006144389**

(43) Date of publication of application:
**28.11.2007 Bulletin 2007/48**

(73) Proprietor: **SYSMEX CORPORATION**
**Kobe-shi,**
**Hyogo 651-0073 (JP)**

(72) Inventors:
• **Kouchi, Yasuhiro**
**Kobe-shi**
**Hyogo 651-0073 (JP)**

• **Saitou, Takeo**
**Kobe-shi**
**Hyogo 651-0073 (JP)**
• **Seike, Masayoshi**
**Kobe-shi**
**Hyogo 651-0073 (JP)**

(74) Representative: **HOFFMANN EITLE**
**Patent- und Rechtsanwälte**
**Arabellastraße 4**
**81925 München (DE)**

(56) References cited:
**WO-A-97/28737        WO-A-02/100266**
**JP-A- 2005 328 924    US-B1- 6 368 272**

**Description**

FIELD OF THE INVENTION

The present invention relates to a biological response prediction system and computer program product of the same.

BACKGROUND

It is extremely important to control the blood glucose level to treat diabetes. Various methods and systems for predicting change in blood glucose level of the patient thus have been conventionally proposed.

Japanese Laid-Open Patent Publication No. 2005-328924 discloses a blood glucose level predicting device for predicting the blood glucose level of a patient using a prediction model. The blood glucose level predicting device stores, in a storing section, a prediction model created based on plurality of history data containing a set of model input values and model output values. The model input values include amount of energy taken or consumed by the patient and physical condition variable values obtained from subjective determination result of the patient regarding his/her physical condition, and the model output values indicate the blood glucose level corresponding to the model input values. The blood glucose level corresponding to an arbitrary predicting condition is predicted using the prediction model of the storing section. According to the blood glucose level predicting device, the model input values necessary in creating the prediction model and predicting the blood glucose level are readily collected by the patient, and thus the blood glucose level may be predicted at high precision not only at medical institutions but also at home without imposing a burden on the patient.

The device disclosed in Japanese Laid-Open Patent Publication No. 2005-328924 uses mathematical formula model or black box prediction model (e.g., model using neural network and model using fuzzy inference base) when creating the prediction model. However, such predicting method is, basically, a method that predicts "the value for this time around to be such value since the relevant value has been indicated in the past," based on the plurality of past history data. Therefore, it is impossible that the method performs a prediction if the input condition differs from the input condition in the history data. Furthermore, it is impossible that the method predicts the blood glucose level at a time point beyond the acquiring time of the history data. That is, the method has a drawback in that the prediction range is limited since the process from the input value to the output value is a "black box" and the method performs the prediction based on the correlation between the input value and the output value.

International Application WO 97/28737A recites in claim 1 a method including formulating an adaptive mathematical model about the behaviour of the patient's blood glucose level, the model taking into account at least the patient's diet, medication and physical strain and comprising comparing the predictive values provided by the model to the measured glucose levels and correcting the mathematical model on the basis of the result of said comparison, and providing the patient with means for utilizing said mathematical model, so that the patient can himself monitor and predict the effect of the treatment he is to follow on the behaviour of his blood glucose level. WO 97/28737 A also recites in claim 2 a monitoring equipment comprising means for receiving data concerning at least the patient's diet, medication and physical strain and for storing the data in a first memory means, data processing means for calculating a predictive value on the basis of the data stored in the first memory means, the predictive value indicating the patient's predictable blood glucose level at a predetermined moment, and corrector means for calculating the difference between the calculated predictive value and the patient's actual blood glucose level calculated at said predetermined moment, and for correcting the mathematical model utilized by the data processing means to calculate a predictive value in order to take into account said difference in the subsequent calculations of predictive values.

SUMMARY

The scope of the present invention is defined solely by the appended claims, and is not affected to any degree by the statements within this summary.

BRIEF DESCRIPTION OF THE DRAWINGS

The first aspect of the present invention relates to a biological response prediction system comprising:

input means for receiving actual measurement data of a subject;
virtual biological organ generating means for generating a virtual biological organ virtually constructed in a computer system using the input actual measurement data, the virtual biological organ corresponding to a function of a biological organ of the subject;

virtual biological response acquiring means for acquiring a virtual biological response indicated by the virtual biological organ when input is provided to the generated virtual biological organ; and

output means for outputting the acquired virtual biological response.

The second aspect of the present invention relates to a method for predicting biological response comprising the steps of:

receiving input of actual measurement data of a subject;

generating virtual biological organ virtually constructed in a computer system using the input actual measurement data, the virtual biological organ corresponding to a function of a biological organ of the subject

acquiring a virtual biological response indicated by the virtual biological organ when input is provided to the generated virtual biological organ; and

outputting the acquired virtual biological response.

The third aspect of the present invention relates to a computer program product for enabling a computer to predict the biological response comprising:

a computer readable medium, and

software instructions, on the computer readable medium, for enabling the computer to perform predetermined operations comprising:

receiving input of actual measurement data of a subject;

generating virtual biological organ virtually constructed in a computer system using the input actual measurement data, the virtual biological organ corresponding to a function of a biological organ of the subject

acquiring a virtual biological response indicated by the virtual biological organ when input is provided to the generated virtual biological organ; and

outputting the acquired virtual biological response.

Fig. 1 is a block diagram showing hardware construction of one embodiment of a biological prediction system;

Fig. 2 is a block diagram showing overall construction of a biological model;

Fig. 3 is a block diagram showing a construction of pancreas model of the biological model;

Fig. 4 is a block diagram showing a construction of a hepatic model of the biological model;

Fig. 5 is a block diagram showing a construction of insulin kinetics model of the biological model;

Fig. 6 is a block diagram showing a construction of a peripheral tissue model of the biological model;

Fig. 7 is a flowchart showing one example of a predicting procedure of the biological response;

Fig. 8 is a flowchart showing procedure of a parameter set generation process;

Fig. 9A is measured OGTT time-series data of blood glucose level;

Fig. 9B is measured OGTT time-series data of blood insulin concentration;

Fig. 10 is a construction view of a template database;

Fig. 11A is template data of blood glucose level;

Fig. 11B is template data of insulin concentration;

Fig. 12A is a diagram showing an error sum of OGTT time-series data against a template of blood glucose level;

Fig. 12B is a diagram showing an error sum of OGTT time-series data against a template of insulin concentration;

Fig. 13A is a view showing time course of glucose absorption rate of when the input condition is for OGTT;

Fig. 13B is a view showing time course of glucose absorption rate of when the input condition is for diet A;

Fig. 14A is a view showing the result of blood glucose level when simulation is performed with the input condition for diet A;

Fig. 14B is a view showing the result of blood insulin concentration when simulation is performed with the input condition for diet A;

Fig. 15A is a view comparing the simulation results of the blood glucose level when the input condition is for OGTT and for diet A;

Fig. 15B is a view comparing the simulation results of the blood insulin concentration when the input condition is for OGTT and for diet A;

Fig. 16 is a flowchart showing another example of a predicting procedure of the biological response;

Fig. 17A is a view showing time-series data of the blood glucose value of the actual measurement OGTT for three hours before administration of medicine;

Fig. 17B is a view showing time-series data of the blood insulin concentration of the actual measurement OGTT for three hours before administration of medicine;

Fig. 18A is a view showing the result of simulation of the blood glucose level;

Fig. 18B is a view showing the result of simulation of the blood insulin concentration;

Fig. 19A is a view showing time-series data of the blood glucose value of the actual measurement OGTT for three hours after administration of medicine;

Fig. 19B is a view showing time-series data of a blood insulin concentration of the actual measurement OGTT for three hours after administration of medicine;

Fig. 20A is a view showing the result of simulation of the blood glucose level;

Fig. 20B is a view showing the result of simulation of the blood insulin concentration;

Fig. 21A is a view comparing the change in predicted blood glucose level before administration and after administration of the medicine; and

Fig. 21B is a view comparing the change in predicted blood insulin concentration before administration and after administration of the medicine.

## DETAILED DESCRIPTION OF THE EMBODIMENT

Embodiments of the biological response system are described hereinafter with reference to drawings.

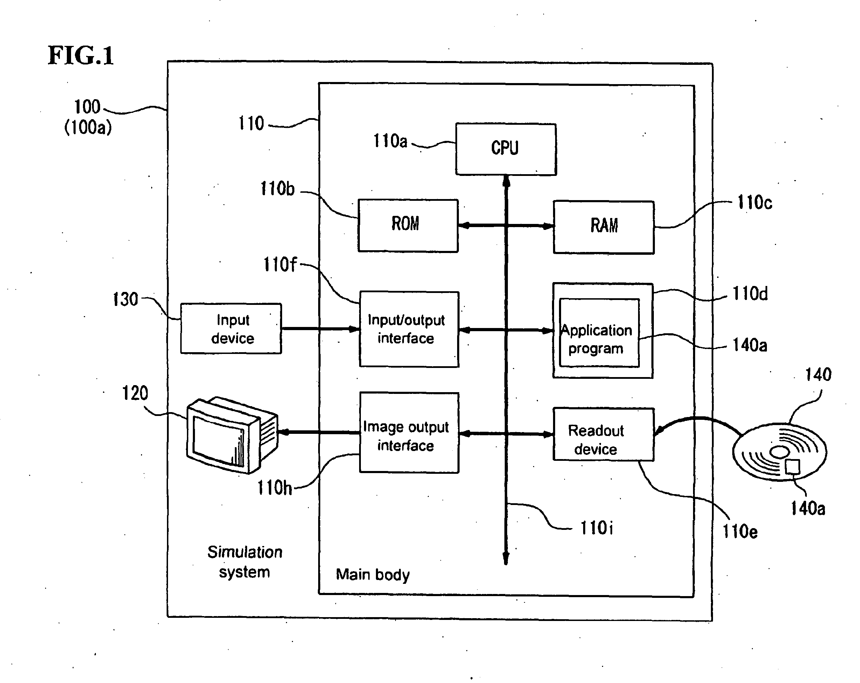

Fig. 1 is a block diagram showing hardware construction of the biological response system. The system 100 is composed of a computer 100a primarily comprising a main body 110, a display 120, and an input device 130. The main body 110 comprises a CPU 110a, a ROM 110b, a RAM 110c, a hard disk 110d, a readout device 110e, an input/output interface 110f, and an image output interface 110h. The CPU 110a, the ROM 110b, the RAM 110c, the hard disk 110d, the readout device 110e, the input/output interface 110f, and the image output interface 110h are data-communicably connected by a bus 110i.

The CPU 110a is capable of executing a computer program recorded in the ROM 110b and a computer program loaded in the RAM 110c. And the CPU 110a executes an application program 140a such as the above programs S2, S3 to realize each function block as described later, thereby the computer functions as the system 100.

The ROM 110b comprises mask ROM, PROM, EPROM, EEPROM, etc. and is recoded with computer programs executed by the CPU 110a and data used for the programs.

The RAM 110c comprises SRAM, DRAM, etc. The RAM 110c is used to read out computer programs recorded in the ROM 110b and the hard disk 110d. And the RAM 110c is used as a work area of the CPU 110a when these computer programs are executed.

The hard disk 110d is installed with an operating system, an application program, etc., various computer programs to be executed by the CPU 110a, and data used for executing the computer programs. The programs S2, S3 are also installed in this hard disk 110d.

The readout device 110e which comprises a flexible disk drive, a CD-ROM drive or DVD-ROM drive is capable of reading out a computer program or data recorded in a portable recording media 140. And the portable recording media 140 stores the application program 140a (S2, S3) to function as a system of the present invention. The computer reads out the application program 140a related to the present invention from the portable recording media 140 and is capable of installing the application program 140a in the hard disk 110d.

In addition to that said application program 140a is provided by the portable recording media 140, said application program 140a may be provided through an electric communication line (wired or wireless) from outside devices which are communicably connected to the computer via said electric communication line. For example, said application program 140a is stored in a hard disk in an application program providing server computer on the Internet to which the computer accesses and said application program 140a may be downloaded and installed in the hard disk 110d.

The hard disk 110d is installed with an operating system which provides a graphical user interface environment, e.g. Windows (Registered trademark) manufactured by US Microsoft Corp. In the explanation hereinafter, the application program 140a (S2, S3) related to this embodiment shall operate on said operating system.

The input/output interface 110f comprises a serial interface, e.g. USB, IEEE1394, RS-232C, etc.; a parallel interface, e.g. SCSI, IDE, IEEE1284, etc.; and an analog interface, e.g. D/A converter, A/D converter, etc. The input/output interface 110f is connected to the input device 130 comprising a keyboard and a mouse and users can input data into the computer using the input data device 130.

The image output interface 110h is connected to the display 120 comprising LCD, CRT or the like so that picture signals corresponding to image data provided from the CPU 110a are output to the display 120. The display 120 displays a picture (screen) based on input picture signals.

[Biological model]

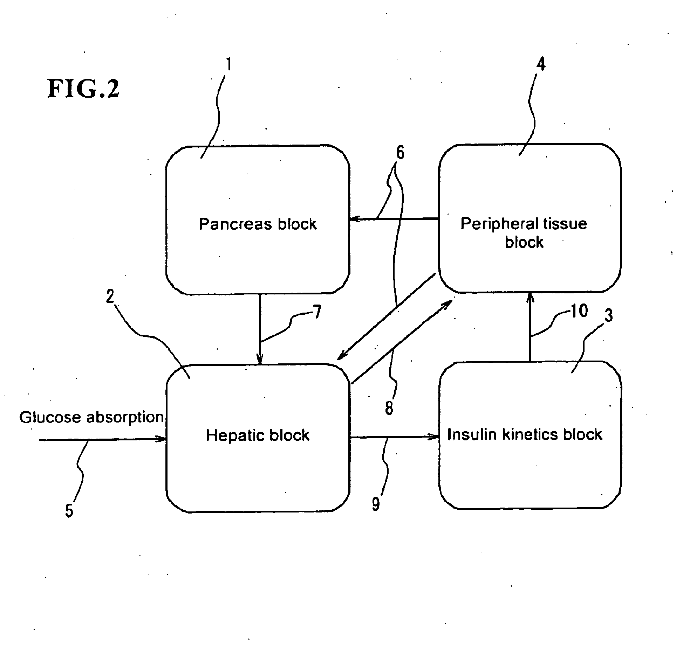

Fig. 2 is a block diagram showing the overall construction of one example of a biological model used in the biological response prediction system 100 according to the present invention. The biological model, in particular, simulates biological organs associated with diabetes, and comprises a pancreas block 1, a hepatic block 2, an insulin kinetics block 3, and a peripheral tissue block 4.

Each block 1, 2, 3, 4 has input and output. As to the pancreas block 1, a blood glucose level 6 is set as input and an insulin secretion rate 7 is set as output to other blocks. As to the hepatic block 2, a glucose absorption 5 from digestive tract, a blood glucose level 6 and an insulin secretion rate 7 are set as input and net glucose release 8 and post liver insulin 9 are set as output to other blocks. As to the insulin kinetics block 3, post liver insulin 9 is set as input and peripheral tissue insulin concentration 10 is set as output to other blocks. As to the peripheral tissue block 4, a net glucose release 8, and insulin concentration 10 in the peripheral tissue are set as input and a blood glucose level 6 is set as output to other blocks.

Glucose absorption 5 is data provided from outside of the biological model. Further, the function blocks 1 to 4 are each realized by the CPU 110a in the biological response prediction system 100 executing the computer program.

Next, the above-mentioned blocks each are described in detail. FGB expresses a fasting blood glucose level (FGB = BG (0)), and Ws expresses an assumed weight. DVg and DVi respectively express a distribution capacity volume against glucose and a distribution capacity volume against insulin.

[Pancreas block of biological model]

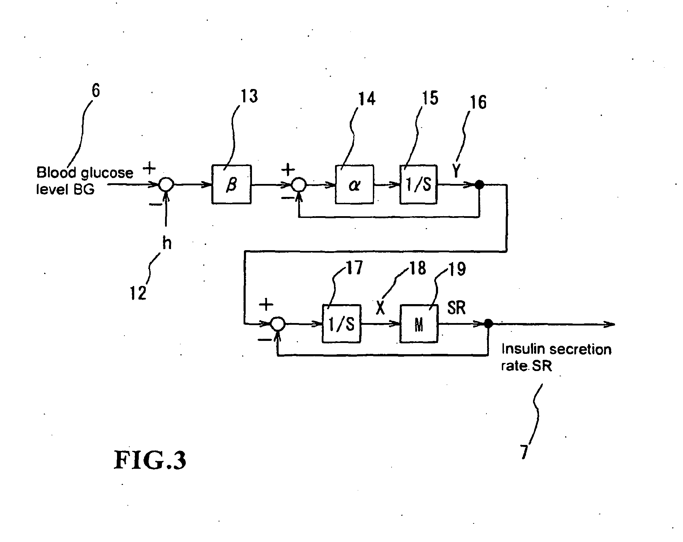

Relationship between input and output of the pancreas block 1 may be expressed using the following differential equation (1) . A block diagram as in Fig. 3 equivalent to the differential equation (1) may be also used.

Differential equation (1):

$$dY/dt = - \alpha\{Y(t) - \beta(BG(t) - h)\} \quad (BG(t) > h)$$
$$= - \alpha Y(t) \quad (BG(t) <= h)$$

$$dX/dt = - M \cdot X(t) + Y(t)$$

$$SR(t) = M \cdot X(t)$$

Variables:

BG(t): blood glucose level
X(t): total amount of insulin capable of secretion from pancreas
Y(t): supply rate of insulin newly supplied for glucose stimulation
SR(t): pancreas insulin secretion rate

Parameters:

h: threshold of glucose concentration capable of stimulating insulin supply
$\alpha$: following performance to glucose stimulation
$\beta$: sensitivity to glucose stimulation
M: secretion rate per unit concentration

where a blood glucose level 6 which is input to the pancreas block in Fig. 2 corresponds to BG(t). The insulin secretion rate 7 which is output of the pancreas block in Fig. 2 corresponds to SR(t).

In a block diagram in Fig. 3, numeral 6 indicates BG (t): blood glucose level; 7 indicates SR (t): pancreas insulin secretion rate from pancreas; 12 indicates h: glucose concentration threshold stimulating insulin supply; 13 indicates $\beta$: glucose stimulation sensitivity; 14 indicates $\alpha$: glucose stimulation following capability; 15 indicates integral element; 16 indicates Y (t) : supply rate of newly supplied insulin to glucose stimulation; 17 indicates integral element; 18 indicates X (t) : total amount of insulin capable of secretion from pancreas; 19 indicates M: secretion rate per unit concentration.

[Hepatic block of biological model]

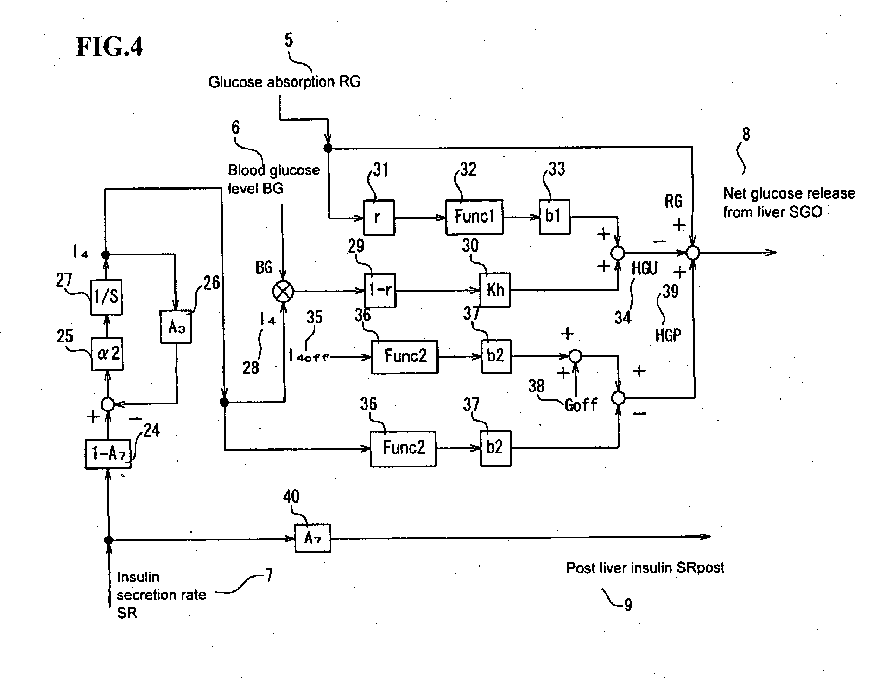

Relationship between input and output of the hepatic block 2 may be described using the following differential equation (2). A block diagram as in Fig. 4 equivalent to the differential equation (2) may be also used.

Differential equation (2):

$$dI_4(t) \ / \ dt \ = \ \alpha 2 \ \{-A_3 I_4(t) \ + \ (1-A_7) \cdot SR(t)\}$$

$$Goff \ (FGB) \ = \ f1 \qquad (FGB \ < \ f3)$$

$$= \ f1 \ + \ f2 \cdot (FGB \ - \ f3) \qquad (FGB \ >= f3)$$

$$Func1 \ (FGB) \ = \ f4 \ - \ f5 \cdot (FGB \ - \ f6)$$

$$Func2 \ (FGB) \ = \ f7 \ / \ FGB$$

$$b1(I_4(t)) \ = \ f8\{1 \ + \ f9 \cdot I_4(t)\}$$

$$HGU(t) \ = \ r \cdot Func1 \ (FBG) \cdot b1(I_4(T)) \cdot RG(t) \ +$$

$$(1-r) \cdot Kh \cdot BG(t) \cdot I_4(t) \qquad (HGU(t) >= \ 0)$$

$$HGP(t) \ = \ I_{4off} \cdot Func2(FBG) \cdot b2 + G_{off}(FBG)$$

$$-I_4(t) \cdot Func2(FBG) \cdot b2 \qquad (HGP(t) >= \ 0)$$

$$SGO(t) \ = \ RG(t) \ + \ HGP(t) \ - \ HGU \ (t)$$

$$SRpost(t) \ = \ A_7 SR(t)$$

Variables:

BG(t): blood glucose level
SR(t): pancreas insulin secretion rate
SRpost(t): post hepatic insulin
RG(t): glucose absorption from digestive tract
HGP(t): hapatic glucose release
HGU (t): hepatic glucose uptake
SGO (t): net glucose from liver
$I_4$ (t) : hepatic insulin concentration

Parameter:

Kh: hepatic glucose uptake rate per unit insulin and unit glucose
$A_7$: insulin uptake rate in liver
Goff: glucose release rate to basal metabolism
b2: adjustment term for hepatic glucose release suppression rate
r: insulin-dependent hepatic glucose uptake distribution rate
$\alpha2$: transmission efficiency to insulin stimulation
$I_{4off}$: insulin concentration threshold of hepatic glucose release suppression

Function:

Goff (FBG): glucose release rate to basal metabolism
Func1 (FBG): hepatic glucose uptake rate to stimulation of glucose from digestive tract
Func2 (FBG) : hepatic glucose release-suppression rate to insulin stimulation
f1 to f9: constants used to express the above-mentioned three elements
$b1(I_4(t))$: adjustment item for hepatic glucose incorporation rate
where the glucose absorption 5 from digestive tract which is input to the hepatic block in Fig. 2 corresponds to RG (t), the blood glucose level 6 to BG (t) and the insulin secretion rate 7 to SR(t). The net glucose release 8 which is output corresponds to SGO (t) and the post liver insulin 9 to SRpost(t).
In a block diagram in Fig. 4, numeral 5 indicates RG (t) : glucose absorption from digestive tract; 6 indicates BG(t): blood glucose level; 7 indicates SR(t): pancreas insulin secretion rate; 8 indicates SGO (t) : net glucose from liver; 9 indicates SRpost (t) : post liver insulin; 24 indicates $(1 - A_7)$ : liver insulin passage rate; 25 indicates $\alpha2$: followability to insulin stimulation; 26 indicates $A_3$: post liver insulin distribution rate; 27 indicates integral element; 28 indicates $I_4$ (t) : hepatic insulin concentration; 9 indicates (1-r): insulin-dependant hepatic glucose incorporation distribution rate; 30 indicates Kh liver insulin-dependent glucose incorporation rate per unit insulin and unit glucose; 31 indicates r: insulin -independent hepatic glucose incorporation rate; 32 indicates Func1 (FGB) : hepatic glucose incorporation rate to glucose stimulation from digestive tract; 33 indicates $b1 (I_4(t))$: adjustment item for hepatic incorporation rate; 34 indicates HGU(t): hepatic glucose incorporation; 35 indicates $I_{4off}$ : insulin concentration threshold of hepatic glucose release inhibition; 36 indicates Func2 (FGB): hepatic release-inhibition rate to insulin stimulation; 37 indicates b2: adjustment items hepatic glucose release-inhibition rate; 38 indicates HGP(t): glucose release rate to basal metabolism; 39 indicates $A_7$: hepatic glucose release; 40, insulin incorporation rate in liver.

[Insulin kinetics block of biological model]

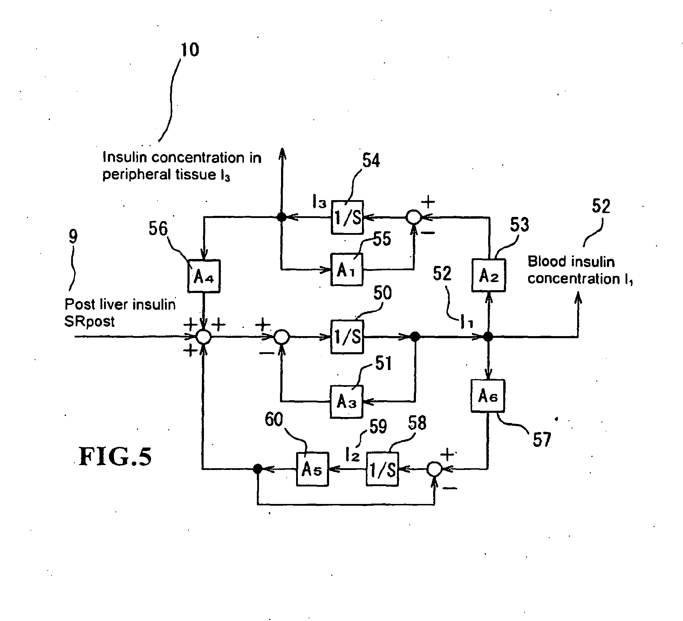

Relationship between input and output of the insulin kinetics secretion may be described using the following differential equation (3). A block diagram as in Fig. 5 equivalent to the differential equation (3) may be also used. Differential equation (3):

$$dI_1(t) / dt = - A_3I_1 (t) + A_5I_2 (t) + A_4I_3 (t) + SRpost(t)$$

$$dI_2(t) / dt = A_6I_1(t) - A_5I_2(t)$$

$$dI_3(t) / dt = A_2I_1(t) - A_1I_3(t)$$

Variables:

SRpost(t): post hepatic insulin
$I_1(t)$: blood insulin concentration
$I_2(t)$: insulin concentration in insulin-independent tissues
$I_3(t)$: insulin concentration in peripheral tissues Parameters:

$A_1$: insulin disappearance rate in peripheral tissues
$A_2$: insulin distribution rate to peripheral tissues
$A_3$: post hepatic insulin distribution rate
$A_4$: post peripheral tissue insulin flow out rate
$A_5$: insulin disappearance rate in insulin-independent tissues
$A_6$: insulin distribution rate to insulin-independent tissues

where the post liver insulin 9 which is input to the insulin kinetics block in Fig. 2 corresponds to SRpost (t). The peripheral tissue insulin concentration 10 which is output corresponds to $I_3(t)$.

In a block diagram in Fig. 5, numeral 9 indicates SRpost (t): post liver insulin; 10 indicates $I_3(t)$: insulin concentration in peripheral tissue; 50 indicates integral element; 51 indicates $A_3$: post liver insulin distribution rate; 52 indicates $I_1(t)$: blood insulin concentration; 53 indicates $A_2$: insulin distribution rate to peripheral tissues; 54 indicates integral element; 55 indicates $A_1$: insulin disappearance rate in peripheral tissue; 56 indicates $A_4$: post peripheral tissue insulin discharge rate; 57 indicates $A_6$: insulin distribution rate to insulin-independent tissue; 58 indicates integral element; 59 indicates $I_2(t)$: insulin concentration in insulin-independent tissue; 60 indicates $A_5$: insulin disappearance rate in insulin-independent tissue.

[Peripheral tissue block of biological model]

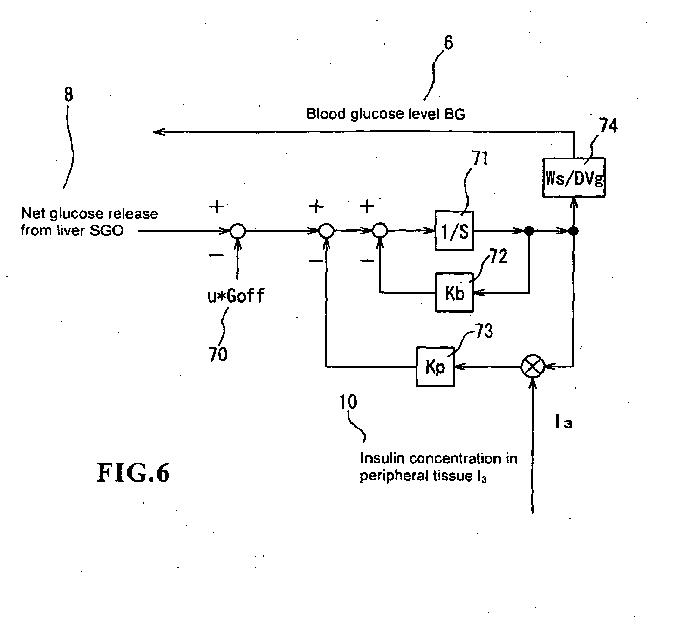

Relationship between input and output of the peripheral tissue block 4 may be described using the following differential equation (4). A block diagram as in Fig. 6 equivalent to the differential equation (4) may be also used.

Differential equation (4):

$$dBG' / dt = SGO(t) - u*Goff (FGB)$$

$$- Kb(BG'(t) - FBG') - Kp \cdot I_3(t) \cdot BG'(t)$$

Variables:

BG' (t) : blood glucose level (BG [mg/dl], BG' [mg/kg])
SGO(t): net glucose from liver
$I_3(t)$ : insulin concentration in peripheral tissues
FBG': fasting blood glucose (provided that FBG'=BG(0))

Parameters:

Kb: insulin-independent glucose consumption rate in peripheral tissues
Kp: insulin-dependent glucose consumption rate in peripheral tissues per unit insulin and per unit glucose
u: ratio of insulin-independent glucose consumption to basal metabolism in glucose release rate to basal metabolism

Functions:

Goff(FGB): glucose release rate to basal metabolism f1 to f3: constant used to express Goff

where the peripheral tissue insulin concentration 10 which is input to the peripheral tissue block in Fig. 2 corresponds to $I_3$ (t), the net glucose 8 from liver corresponds to SGO(t). The blood glucose level 6 which is output corresponds to BG(t).
In a block diagram in Fig. 6, numeral 6 indicates BG (t) : blood glucose level; 8 indicates SGO(t): net glucose from liver; 10 indicates $I_3$(t): insulin concentration in peripheral tissues; 70 indicates u*Goff(FGB): insulin-independent glucose consumption rate to basal metabolism; 71 indicates integral element; 72 indicates Kb: insulin-independent glucose consumption rate in peripheral tissues; 73 indicates Kp: insulin-dependent glucose consumption rate in peripheral tissues per unit insulin and per unit glucose; 74 indicates Ws/DVg: unit conversion constant.
As shown in Fig. 2, since inputs and outputs are mutually connected between the blocks constituting the present system, by giving glucose absorption 5 from digestive tract, it is possible to calculate and simulate time courses of blood glucose level and insulin concentration based on the mathematical formula.
With regard to calculation of the differential equations of the present system, e.g. E-Cell (software disclosed by Keio University) and MatLab (manufactured by The MathWorks, Inc.) may be employed. Or other calculation system may be employed.

[Predicting procedure of biological response]

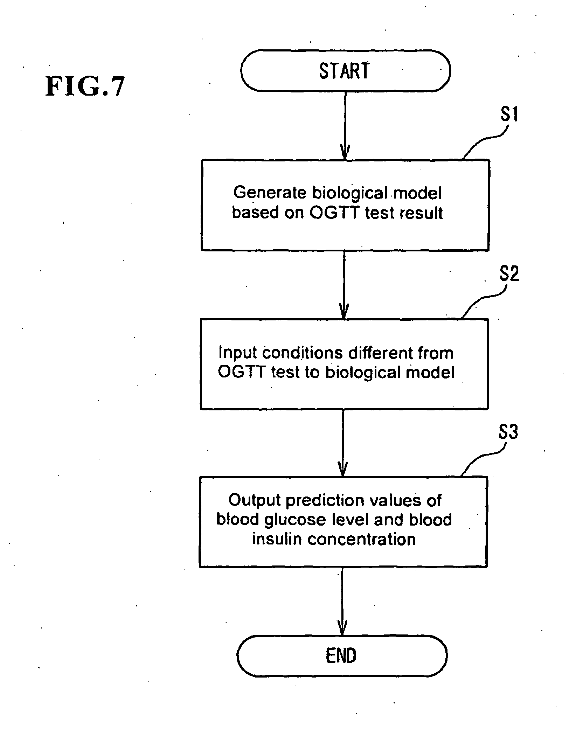

Fig. 7 shows one example of the predicting procedure of the biological response using the present system 100. First, OGTT (Oral Glucose Tolerance Test) is actually performed on the subject (patient). When the result of the OGTT is input to the system 100, the system 100 simulates biological organs associated with diabetes of the subject, and generates a biological model (see Fig. 2) in which the functions of the biological organs are represented by a mathematical model (step S1).
In obtaining a simulated biological response using the obtained biological model, conditions different from those the OGTT used in diagnosis are input, and change in blood glucose level and blood insulin concentration is predicted (step S2). The change in blood glucose level etc. of when taking a predefined diet can be predicted since the change in blood glucose level etc. under conditions different from the conditions of OGTT can be predicted, whereby data beneficial in treating (caring) diabetes are obtained. Here, predefined diet is a diet in which content and consuming condition (speed of eating etc.) are controlled so that the glucose absorption rate at the intestine of the subject is a predefined value.
The system 100 then performs simulation based on the input conditions, and outputs prediction values of the blood glucose level and the blood insulin concentration (step S3) .

[Generation of biological model]

The biological model generation step (step S1) described above will now be described in detail.
When the result of the OGTT performed on the subject is input to the system 100, the biological model (see Fig. 2) stimulating biological organs associated with diabetes of the subject is generated in the present system 100.
To simulate the biological organs of individual subject using the above-mentioned biological models as shown in Figs. 2 to 6, it is required to generate a biological model having characteristics suited for individual subject. To be more specific, it is required to determine parameters and initial values of variables of biological model according to the individual subject, and apply the determined parameters and initial values to the biological model, thereby generating a biological model suited for the individual subject. (Unless otherwise specified, an initial value of variable is also included in parameters to be generated.)
The present system thus has a function of obtaining parameter set or a set of parameters of the biological model (hereinafter simply referred to also as "parameter set") by a parameter set generating section, and generating the biological model applied with the obtained parameter set. This function is also realized by a computer program.

The parameter set generated by the parameter set generating section is applied to the biological model, and a biological model calculating section (virtual biological response acquiring section) of the system simulates the function of the biological organs and outputs the simulated response simulating the actual biological response (test result).

[Parameter set generating section]

In the following, description will be made for a parameter set generating section for forming a biological model that simulates a biological organ of a subject based on an actual OGTT result (biological response) of the subject (biological body). OGTT is a test of measuring the blood glucose level and the blood insulin concentration by orally taking glucose and taking blood a few times after a predetermined time has elapsed, and such test is actually frequently carried out since the load on the subject is small compared to glucose clamp.

[OGTT time-series data input: Step S1-1]

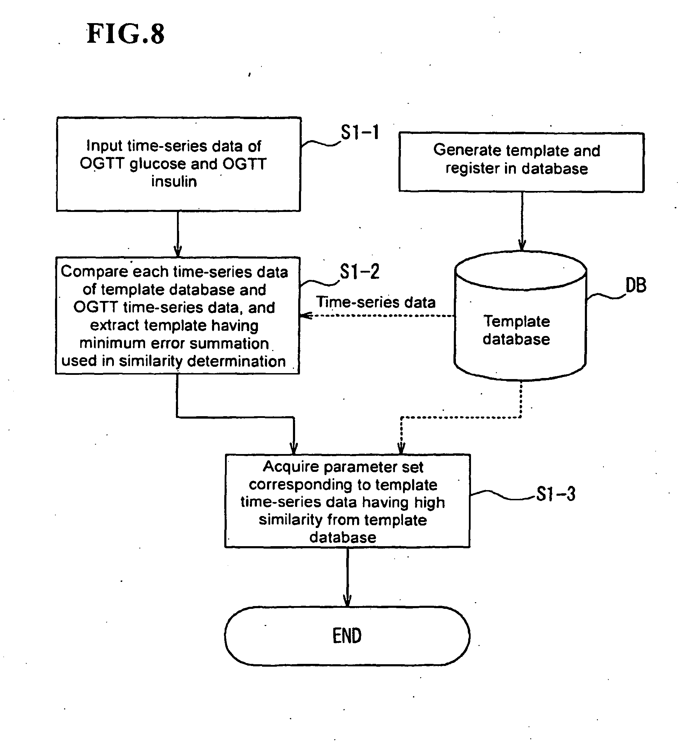

Fig. 8 is a flowchart showing procedures in which the parameter set generating section of system 100 obtains a parameter set of the biological model. In order to obtain parameters, first, an inputting step of OGTT (oral glucose tolerance test) time-series data which is an actual test result (biological response) is executed as shown in the flowchart (Step S1-1).

OGTT time-series data are a result of OGTT (given amount of glucose solution is orally loaded to measure the time-series of blood glucose level and blood insulin concentration) from the actual examination of subject simulated by a biological model. Here, two data of and OGTT glucose data (blood glucose change data) and OGTT insulin (blood insulin concentration change data) are input as OGTT time-series data.

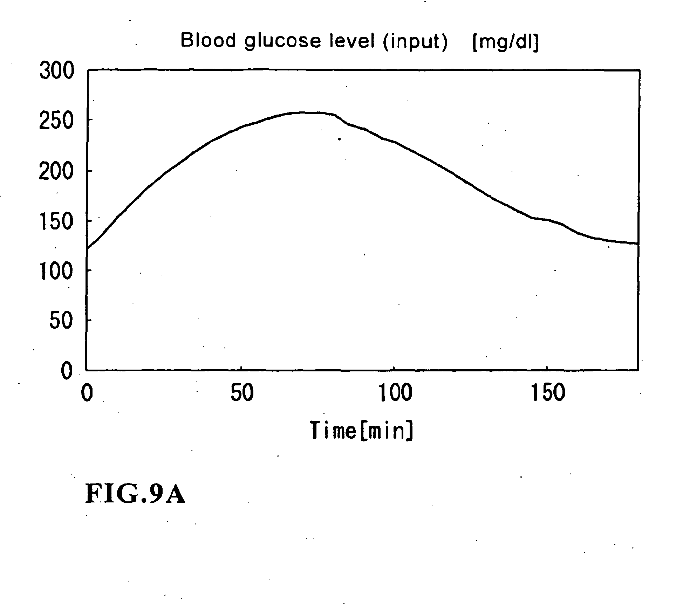

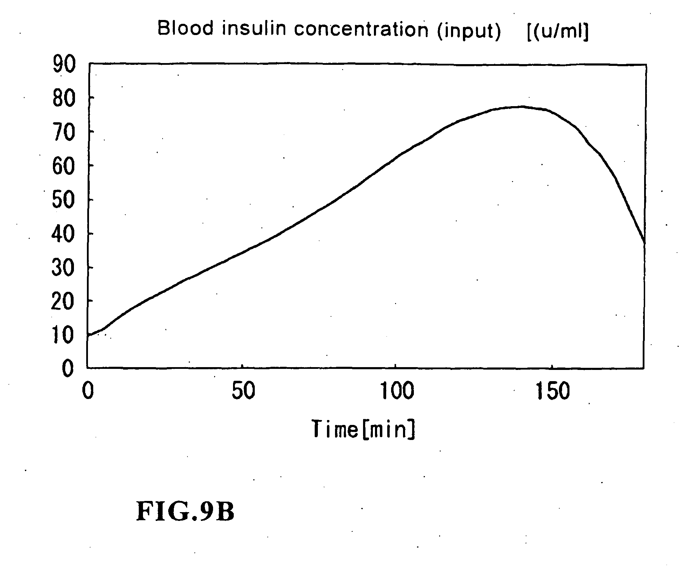

Fig. 9A shows an example of the blood glucose level change data and Fig. 9B shows an example of the blood insulin concentration change data as OGTT time-series data to be input. In Fig. 9A, the blood glucose level change data is measured data corresponding to time course of blood glucose level BG (t), one of output items in the biological model shown in Figs. 2 to 6. In Fig. 9B, the blood insulin concentration change data is measured data corresponding to time course of blood insulin concentration $I_1$ (t), one of output items in the biological model shown in Figs. 2 to 6.

[Template matching: Step SI-2]

Next, the present system 100 matches the input OGTT time-series data to the template of template database DB.

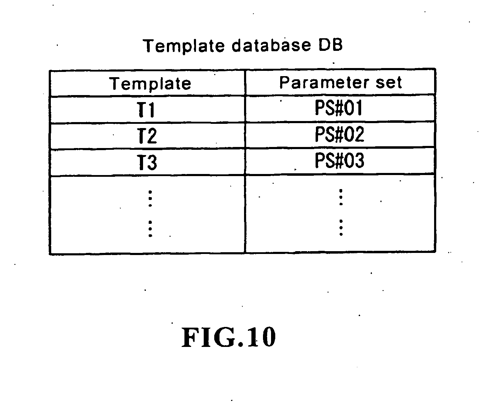

As shown in Fig. 10, the template database DB is preliminarily stored with a plurality sets of data, which are biological model reference output values T1, T2, ... as a template and parameter setPS#01, PS#02 ... correspondent to the reference output value to generate the reference output value. To make up a pair of reference output value and parameter set, a random reference output value is assigned by an appropriate parameter set, or on the contrary, a biological model output at the time when a random parameter set is selected is obtained by the biological simulation.

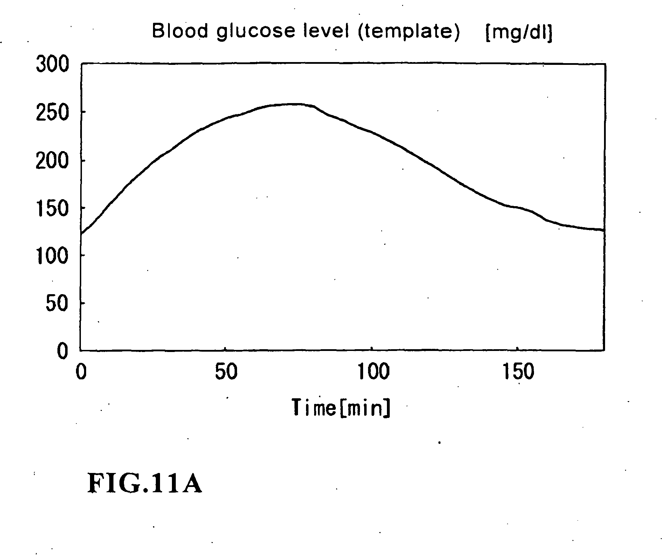

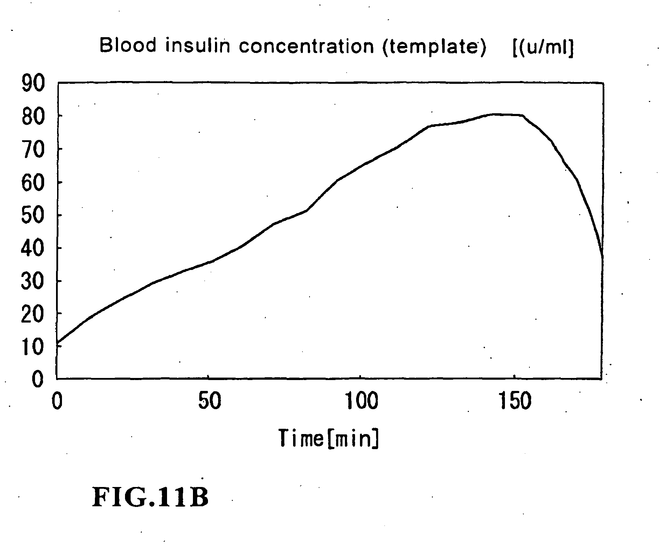

Fig. 11A and Fig. 11B shows examples of the template (reference output value) T1. Fig. 11A is a blood glucose change data as a template, which is reference time-series data corresponding to time course of the blood glucose level BG(t), one of output items in the biological model shown in Figs. 2 to 6. Fig. 11B is blood insulin concentration change data as a template, which is reference time-series data corresponding to time course of blood insulin concentration $I_1$ (t), one of output items in the biological model shown in Figs. 2 to 6.

The system 100 computes similarity between each reference time-series datum of the above-mentioned template database DB and OGTT time-series data. The similarity is obtained by obtaining error summation. The error summation is obtained by the following formula.

$$\text{Error summation} = \alpha\Sigma|BG(0)-BGt(0)|+\beta\Sigma|PI(0)-PIt(0)|$$

$$+\alpha\Sigma|BG(1)-BGt(1)|+\beta\Sigma|PI(1)-PIt(1)|$$

$$+\alpha\Sigma|BG(2)-BGt(2)|+\beta\Sigma|PI(2)-PIt(2)|$$

$$+\ldots$$

$$=\alpha\{\Sigma|BG(t)-BGt(t)|\}+\beta\{\Sigma|PI(t)-PIt(t)|\}$$

where

BG: input data blood glucose level [mg/dl]
PI: input data blood insulin concentration [μU/ml]
BGt: template blood glucose level[mg/dl]
PIt: template blood insulin concentration [μU/ml]
t: time[minute]
Here, α and β are coefficient used for normalization

$$\alpha = 1/\text{Average} \{\Sigma BG(t)\}$$

$$\beta = 1/\text{Average} \{\Sigma PI(t)\}$$

The average of the formula shows average level to all templates stored in the template database DB.

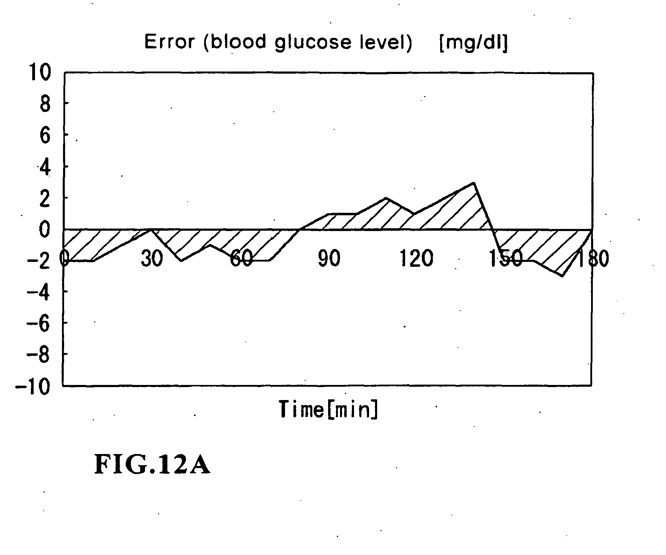

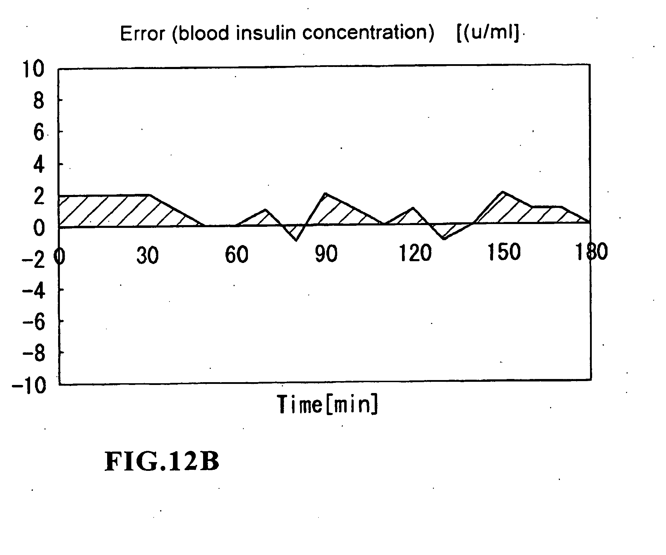

Fig. 12A and Fig. 12B show the OGTT time-series error summation (no normalization) to the template T1. More specifically, Fig. 12A shows an error between the blood glucose level of Fig. 9A and the blood glucose level of Fig. 11A. Fig. 12B shows an error between the blood insulin concentration of Fig. 9B and the blood insulin concentration of Fig. 11B.
Based on Fig. 9A and Fig. 9B input data (date in the range of 0 to 180 minutes every 10 minutes) and Fig. 11A and Fig.11 B template T1,

$$\Sigma | BG(t) - BGt(t) | = 29$$

$$\Sigma | PI(t) - PIt(t) | = 20$$

where, provided α= 0.00035, β = 0.00105

$$\text{error summation} = (0.00035 \times 29) + (0.00105 \times 20)$$

$$= 0.03115$$

Thus, CPU 100a obtains an error summation to each template in the template database DB, and determines the template having the minimum error summation (similarity). Thus, CPU 100a determines the template which is the most approximate to OGTT time-series data (Step S1-2).

[Acquisition of parameter set: Step 1-3]

Further, in a step S1-3, the system 100 obtains from template database DB a parameter set corresponding to the template which has been determined in the step SI-2 and has been judged to be similar in the step S1-3. That means, a parameter set PS#01 corresponding to the template T1 is obtained (Refer to Fig. 10) .
The table below exemplifies the specific numeral values of the parameter values included in the parameter set PS#01 obtained by the above-mentioned way.

Parameter set PS#01 corresponding to template T1

|  | Parameter | Value | Unit |
|---|---|---|---|
| Pancreas | h | 92.43 | [mg/dl] |
| | $\alpha$ | 0.228 | [l/min] |
| | $\beta$ | 0.357 | $[(\mu U/ml) \cdot (dl/mg) \cdot (1/min)]$ |
| | M | 1 | [l/min] |
| | X (0) | 336.4 | $[\mu U/ml]$ |
| | Y (0) | 4.4 | $[(\mu U/ml) \cdot (1/min)]$ |
| Insulin kinetics | $A_1$ | 0.025 | [l/min] |
| | $A_2$ | 0.042 | [l/min] |
| | $A_3$ | 0.435 | [l/min] |
| | $A_4$ | 0.02 | [l/min] |
| | $A_5$ | 0.394 | [l/min] |
| | $A_6$ | 0.142 | [l/min] |
| Peripheral tissues | Kb | 0.009 | [l/min] |
| | Kp | 5.28E-05 | $[(ml/\mu U) \cdot (1/min)]$ |
| | u | 0.6 | |
| Liver | $A_7$ | 0.47 | |
| | Kh | 0.0000462 | $[(ml/\mu U) \cdot (l/min) \cdot (dl/kg)]$ |
| | b2 | 1.1 | |
| | r | 0.98 | |
| | $\alpha 2$ | 0.228 | |
| | $|_{4\ off}$ | 5 | $[\mu U/ml]$ |

The method of generating the parameter set (biological model) is not limited to template matching as described above. For instance, the parameter set may be generated through genetic algorithm. That is, genetic algorithm may be applied in generating the parameter set, where an initial group of parameter set is produced at random, and selection/ chiasm/mutation process is performed on the parameter set (individual) contained in the initial group to generate a new child group. In the method of generating the parameter set through genetic algorithm, the parameter set that outputs the simulated response close to the input biological response (test result) is used among the generated parameter sets. Thus, the specific generating method of the biological model generating section is not particularly limited as long as the biological model that outputs simulated response simulating the input biological response can be generated.

[Prediction of biological response]

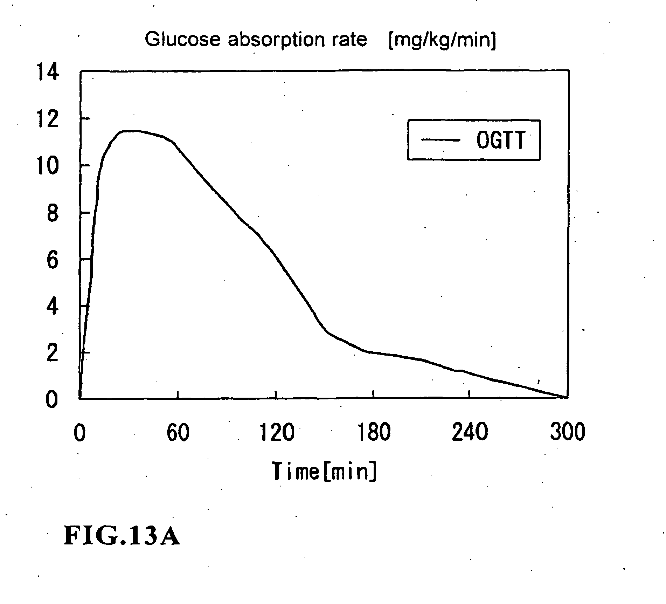

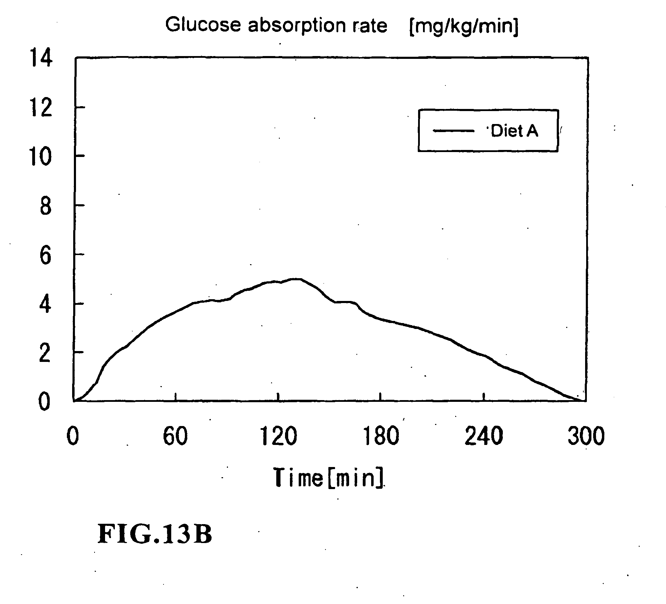

Simulation is performed using the biological model obtained as above to predict the biological response of the subject, that is, time course of blood glucose level and blood insulin concentration. In the present example, simulation is performed with the input condition changed from OGTT to diet A (hypothetical value) to predict the change in blood glucose level and blood insulin concentration. Fig. 13 shows the difference in glucose absorption rate in the intestine between OGTT and diet A, in other words, the difference in amount of glucose, which is the input condition to the biological model. In the present biological response prediction system 100, the output of the biological model can be obtained even if the input condition to the biological model is changed within a constant range and the biological response corresponding to various states and conditions can be predicted since the parameter set configuring the biological model is obtained as described above.

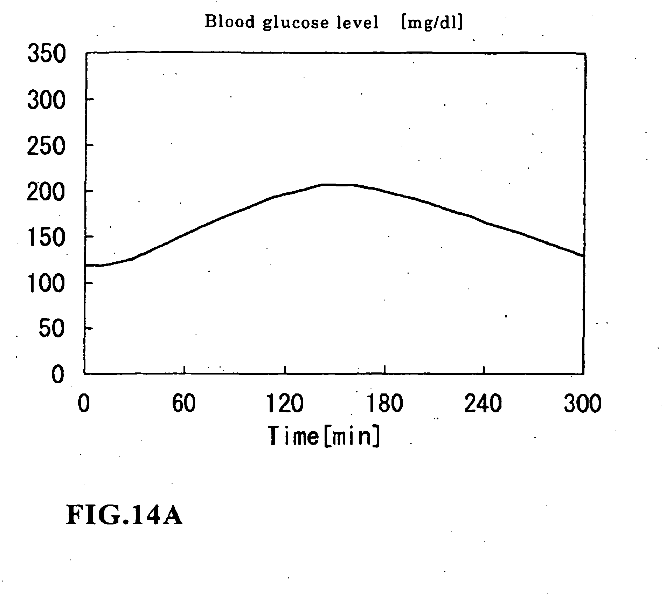

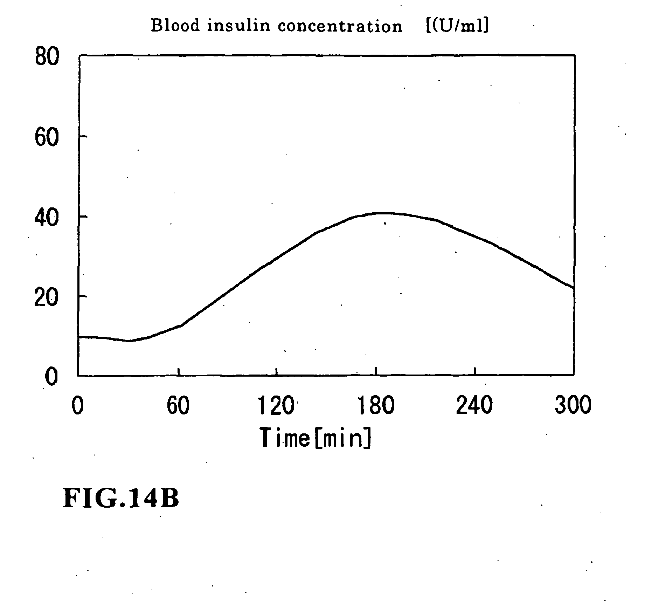

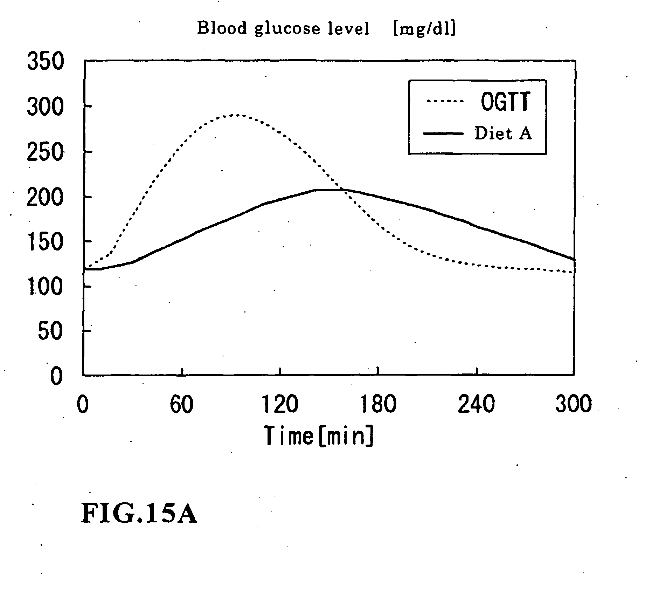

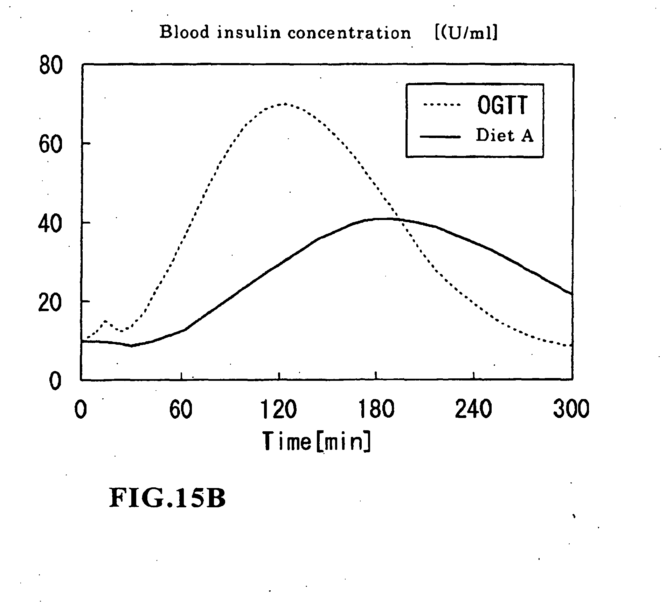

Fig. 14 shows the time course of the blood glucose level and the blood insulin concentration of when the values shown in Fig. 13B are input to the biological model and simulation is performed, and Fig. 15 shows the difference in output of when the input condition is for OGTT and for diet A.

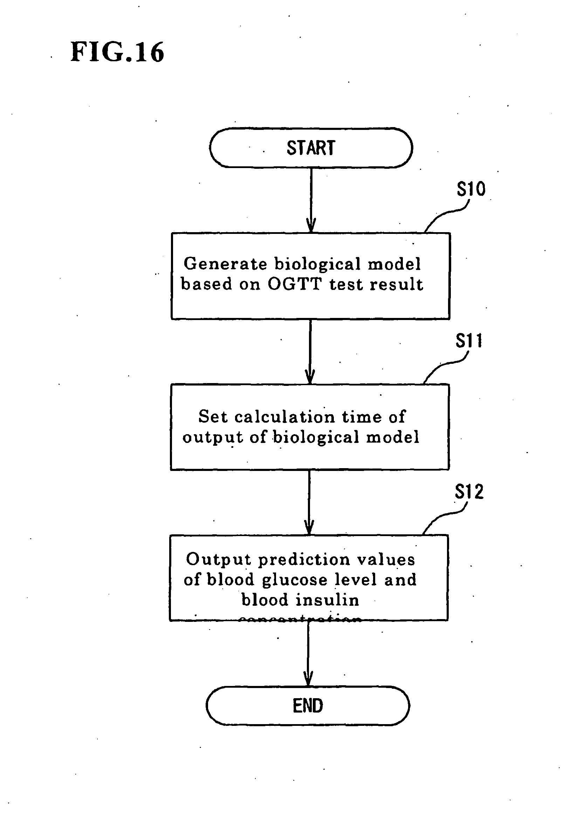

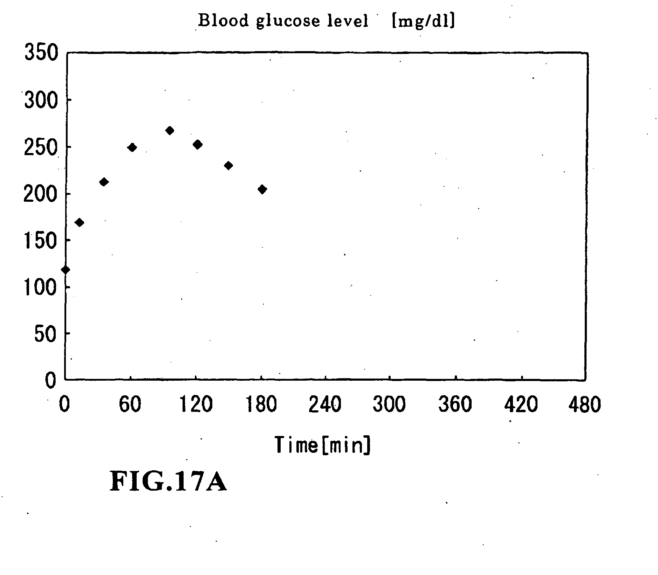

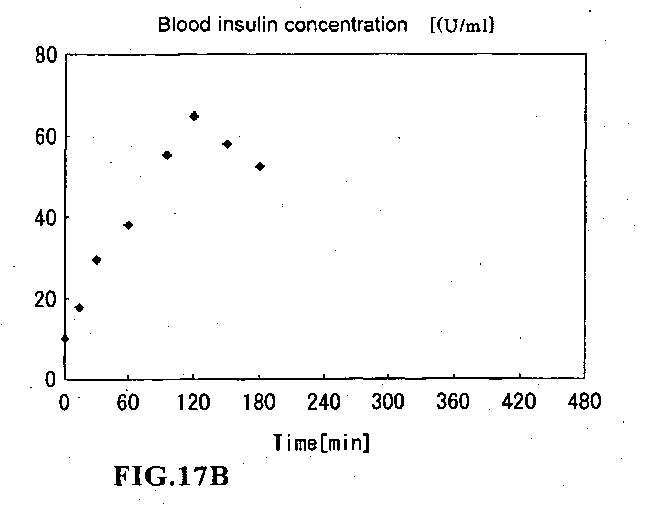

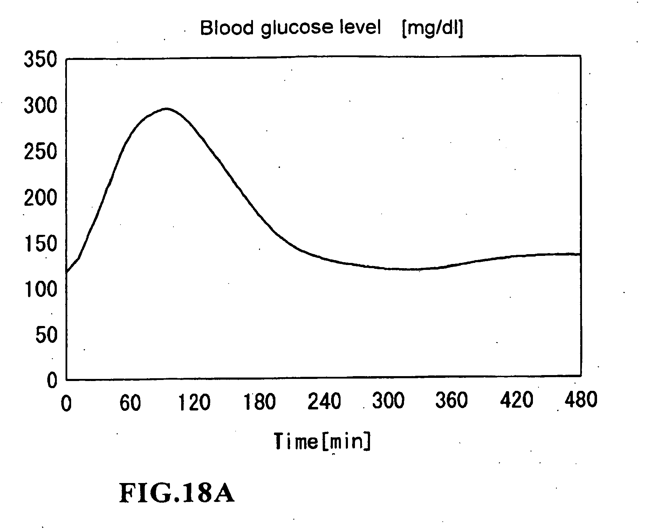

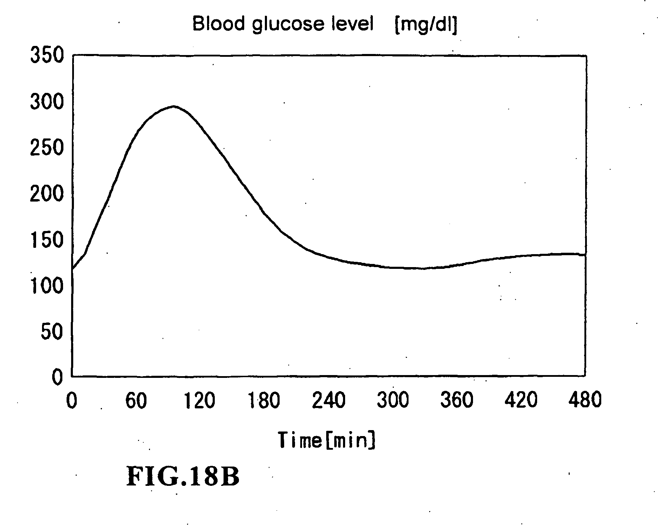

Fig. 16 is a flowchart showing another example of the predicting procedure of the biological response, where step S10 and step S12 are the same as step S1 and step S3 in Fig. 7, but the calculation time of the biological model calculating section to calculate the output of the biological model based on the parameter set configuring the biological model by the calculating time setting means is set in this example (step S11). When desiring to make the prediction until eight hours elapse from the start of glucose load, the calculating time is set (input) to "eight hours" by the calculating time setting means including keyboard and mouse. Figs. 17A and 17B are actual measurement OGTT time-series data for three hours, where Fig. 17A shows the time-series data for blood glucose level and Fig. 17B shows the time-series data for blood insulin concentration. Figs. 18A and 18B show the simulation of change in blood glucose level and blood insulin concentration until eight hours elapse from the start of glucose load using the biological model created based on the data shown in Figs. 17A and 17B. The values shown in Figs. 18A and 18B are calculated using the parameter set configuring the biological model, whereby the blood glucose level and the blood insulin concentration can be quantitatively predicted.

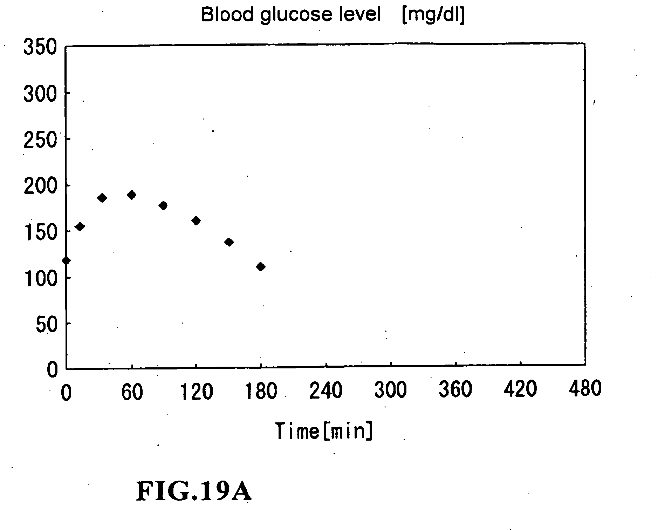

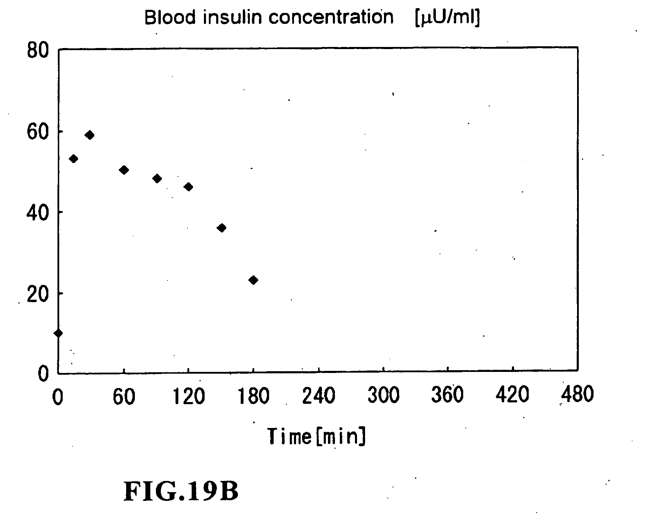

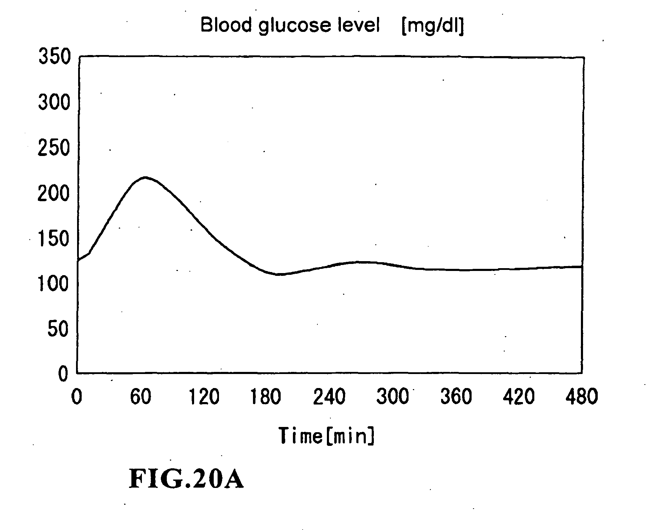

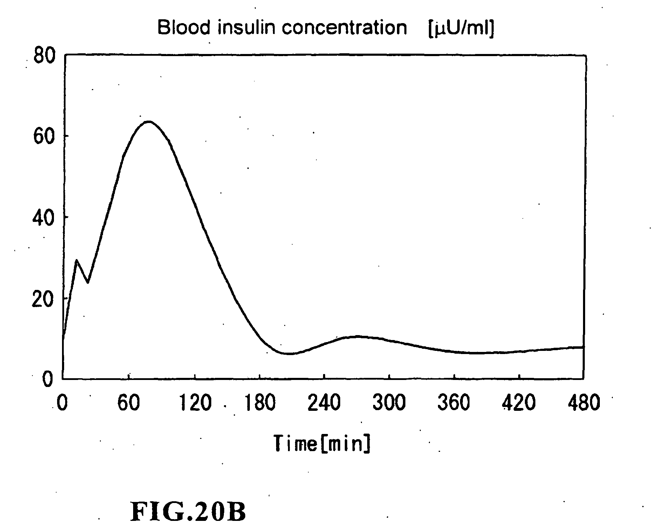

According to the system of the present invention, the prediction of biological response under the condition different from when simply having glucose as the input condition such as when administering a predetermined medicine along with glucose can be performed. Figs. 19A and 19B are actual measurement OGTT time-series data for three hours of when medicine (Nateglinide®) is administered to the subject. Fig. 19A shows the time-series data for the blood glucose level, and Fig. 19B shows the time-series data for the blood insulin concentration. Another parameter set different from the parameter set obtained based on the data of Figs. 17A and 17B is obtained with the relevant data. Figs. 20A and 20B show the result of simulating the change in blood glucose level and blood insulin concentration until eight hours elapse from the start of glucose load using the other parameter set. In this case as well, the blood glucose level and the blood insulin concentration under administration of medicine can be quantitatively predicted since calculation is performed using the parameter set configuring the biological model.

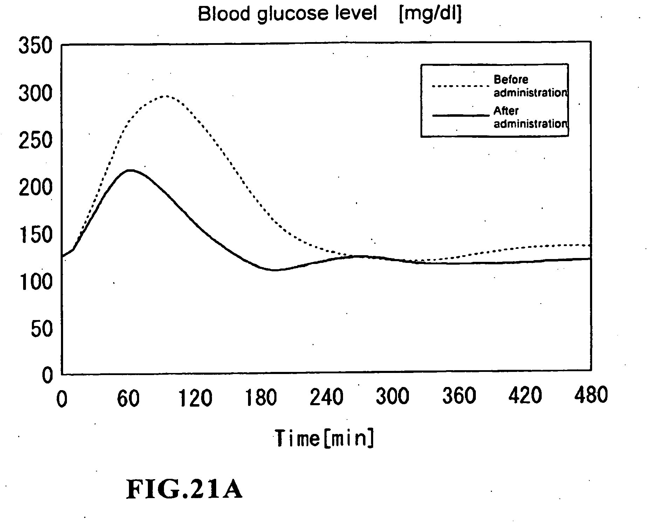

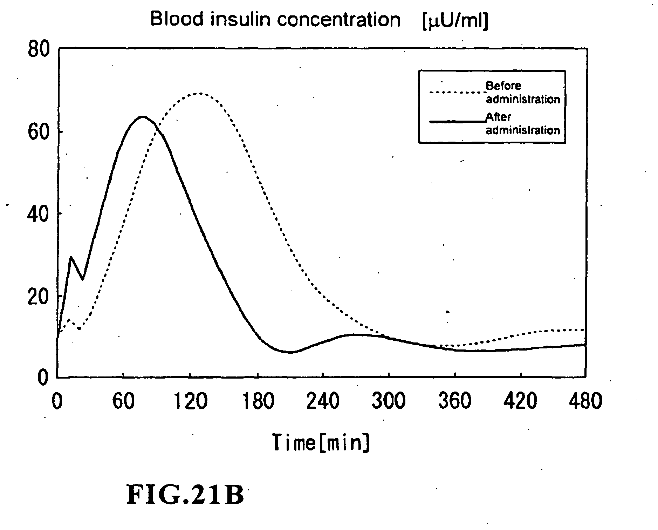

Figs. 21A and 21B are views comparing the change in predicted biological response before administration and after administration of the medicine. Fig. 21A shows the time-series data for the blood glucose level, and Fig. 21B shows the time-series data for the blood insulin concentration. As apparent from Figs. 21A and 21B, how the blood glucose level and the blood insulin concentration change over time by administering medicine to the subject can be quantitatively predicted. Therefore, the effect of treatment of the medicine can be quantitatively predicted to a certain extent and an effective diabetes treatment can be performed.

Similarly, the change in blood glucose level and blood insulin concentration can be quantitatively predicted even if medicine other than Nateglinide is administered.

The foregoing detailed description and accompanying drawings have been provided by way of explanation and illustration, and are not intended to limit the scope of the appended claims. Many variations in the presently preferred embodiments illustrated herein will be obvious to one of ordinary skill in the art, and remain within the scope of the appended claims and their equivalents.

**Claims**

1. A biological response prediction system comprising:

    input means (130) for receiving actual measurement data of a subject;
    virtual biological organ generating means (110a) for generating a virtual biological organ virtually constructed in a computer system using the input actual measurement data, the virtual biological organ corresponding to a function of a biological organ of the subject;
    virtual biological response acquiring means (110a) for acquiring a virtual biological response indicated by the virtual biological organ when input is provided to the generated virtual biological organ; and
    output means (110a , 120) for outputting the acquired virtual biological response.

2. The biological response prediction system according to Claim 1, further comprising:

    input value setting means for setting an input value to the virtual biological organ; wherein
    the virtual biological response acquiring means (110a) is configured to acquire the virtual biological response of the virtual biological organ to which the input value set by the input value setting means is provided.

3. The biological response prediction system according to Claim 1 or 2, further comprising:

    acquiring time period setting means for setting a time period to acquire the virtual biological response of the virtual biological organ; wherein

the virtual biological response acquiring means (110a) is configured to acquire the virtual biological response indicated by the virtual biological organ in the time period set by the acquiring time period setting means.

4. The biological response prediction system according to any one of Claim 1 to 3, wherein
the virtual biological organ includes a block (1,2,3,4) indicating the function of the biological organ associated with metabolism of glucose and/or insulin; and
the virtual biological response acquiring means (110a) is configured to compute the virtual biological response using the virtual biological organ based on the input value of the block (1,2,3,4).

5. The biological response prediction system according to any one of Claim 1 to 4, further comprising:

a memory (140) for storing a mathematical model that comprises a plurality of parameters and represents a function of a biological organ, wherein
the virtual biological organ generating means (110a) is configured to generated a plurality of parameter values of the mathematical model by using the actual measurement data of the subject, the mathematical model to which the parameter values are applied representing the function of the biological organ of the subject.

6. The biological response prediction system according to Claim 5, wherein
the virtual biological organ generating means (110a) includes:

acquiring means for acquiring a plurality of sets of parameter values;
comparing means for comparing each virtual biological response generated when the plurality of sets of parameter values acquired by the acquiring means is applied to the mathematical model, with the actual measurement data of the subject received by the input means; and
selecting means for selecting one of the plurality of sets of parameter values acquired by the acquiring means based on the comparison result of the comparing means.

7. The biological response prediction system according to Claim 6, wherein
the selecting means is configured to select the set of parameter values corresponding to the virtual biological response having the highest matching rate with respect to the factual measurement data of the subject.

8. The biological response prediction system according to Claim 6 or 7, further comprising:

template storing means for storing a plurality of sets of parameter values and a plurality of template data representing the virtual biological responses, wherein each of the virtual biological responses represented by each of the template data corresponds to each of the sets of parameter values,

wherein the acquiring means is configured to read the set of parameter values and the template data from the template storing means.

9. The biological response prediction system according to Claim 6 or 7, wherein
the acquiring means includes,

initial group generating means for generating a plurality of initial groups of the parameter value; and
genetic algorithm executing means for executing genetic algorithm on the plurality of initial groups of the parameter value generated by the initial group generating means, and generating a plurality of sets of parameter values.

10. A method for predicting biological response comprising the steps of:

receiving input of actual measurement data of a subject;
generating virtual biological organ virtually constructed in a computer system using the input actual measurement data, the virtual biological organ corresponding to a function of a biological organ of the subject
acquiring a virtual biological response indicated by the virtual biological organ when input is provided to the generated virtual biological organ; and
outputting the acquired virtual biological response.

11. A computer program product for enabling a computer to predict the biological response comprising:

a computer readable medium, and
software instructions, on the computer readable medium, for enabling the computer to perform predetermined operations comprising:

receiving input of actual measurement data of a subject;
generating virtual biological organ virtually constructed in a computer system using the input actual measurement data, the virtual biological organ corresponding to a function of a biological organ of the subject
acquiring a virtual biological response indicated by the virtual biological organ when input is provided to the generated virtual biological organ; and
outputting the acquired virtual biological response.

**Patentansprüche**

1. Vorhersagesystem einer biologischen Reaktion, umfassend:

ein Eingabemittel (130) zum Aufnehmen aktueller Messdaten eines Subjekts;
ein Erzeugungsmittel eines virtuellen biologischen Organs (110a) zum Erzeugen eines virtuellen biologischen Organs, das virtuell in einem Computersystem unter Verwendung der eingegebenen aktuellen Messdaten konstruiert wird, wobei das virtuelle biologische Organ einer Funktion eines biologischen Organs des Subjekts entspricht;
ein Erlangungsmittel einer virtuellen biologischen Reaktion (110a) zum Erlangen einer virtuellen biologischen Reaktion, die durch das virtuelle biologische Organ angezeigt wird, wenn eine Eingabe dem erzeugten virtuellen biologischen Organ zugeführt wird; und
ein Ausgabemittel (110h, 120) zum Ausgeben der erlangten virtuellen biologischen Reaktion.

2. Vorhersagesystem einer biologischen Reaktion nach Anspruch 1, ferner umfassend:

ein Eingabewerteinstellmittel zum Einstellen eines Eingabewerts für das virtuelle biologische Organ; wobei
das Erlangungsmittel der virtuellen biologischen Reaktion (110a) eingerichtet ist, die virtuelle biologische Reaktion des virtuellen biologischen Organs zu erlangen, dem der durch das Eingabewerteinstellmittel eingestellte Eingabewert zugeführt wird.

3. Vorhersagesystem einer biologischen Reaktion nach Anspruch 1 oder 2, ferner umfassend:

ein Einstellmittel eines Erlangungszeitraums zum Einstellen eines Zeitraums, um die virtuelle biologische Reaktion des virtuellen biologischen Organs zu erlangen; wobei
das Erlangungsmittel der virtuellen biologischen Reaktion (110a) eingerichtet ist, um die durch das virtuelle biologische Organ in dem durch das Einstellmittel des Erlangungszeitraums eingestellten Zeitraum angezeigte biologische Reaktion zu erlangen.

4. Vorhersagesystem einer biologischen Reaktion nach einem der Ansprüche 1 bis 3, wobei
das virtuelle biologische Organ einen Block (1, 2, 3, 4) enthält, der die Funktion des biologischen Organs anzeigt, das mit einem Stoffwechsel von Glucose und/oder Insulin verbunden ist; und
das Erlangungsmittel der virtuellen biologischen Reaktion (110a) eingerichtet ist, die virtuelle biologische Reaktion unter Verwendung des virtuellen biologischen Organs auf der Grundlage des Eingabewerts des Blocks (1, 2, 3, 4) zu berechnen.

5. Vorhersagesystem einer biologischen Reaktion nach einem der Ansprüche 1 bis 4, ferner umfassend:

einen Speicher (140) zum Speichern eines mathematischen Modells, das eine Mehrzahl von Parametern aufweist und eine Funktion eines biologischen Organs repräsentiert, wobei
das Erzeugungsmittel des virtuellen biologischen Organs (110a) eingerichtet ist, eine Mehrzahl von Parameterwerten des mathematischen Modells unter Verwendung der aktuellen Messdaten des Subjekts zu erzeugen, wobei das mathematische Model, dem die Parameterwerte zugeführt werden, die Funktion des biologischen Organs des Subjekts repräsentiert.

6. Vorhersagesystem einer biologischen Reaktion nach Anspruch 5, wobei

das Erzeugungsmittel des virtuellen biologischen Organs (110a) enthält:

ein Erlangungsmittel zum Erlangen einer Mehrzahl von Sätzen von Parameterwerten;
ein Vergleichsmittel zum Vergleichen jeder biologischen Reaktion, die erzeugt wird, wenn die Mehrzahl von Sätzen von Parameterwerten, die durch das Erlangungsmittel erlangt wurden, dem mathematischen Modell zugeführt wird, mit den aktuellen Messdaten des Subjekts, die von dem Eingabemittel aufgenommen wurden; und
ein Auswahlmittel zum Auswählen eines der Mehrzahl von Sätzen von Parameterwerten, die durch das Erlangungsmittel auf der Grundlage des Vergleichsergebnisses des Vergleichsmittels erlangt wurden.

7.  Vorhersagesystem einer biologischen Reaktion nach Anspruch 6, wobei
das Auswahlmittel eingerichtet ist, den Satz von Parameterwerten auszuwählen, welcher der virtuellen biologischen Reaktion mit dem höchsten Übereinstimmungsgrad in Bezug auf die aktuellen Messdaten des Subjekts entspricht.

8.  Vorhersagesystem einer biologischen Reaktion nach Anspruch 6 oder 7, ferner umfassend:

ein Vorlagenspeichermittel zum Speichern einer Mehrzahl von Sätzen von Parameterwerten und einer Mehrzahl von Vorlagendaten, welche die virtuellen biologischen Reaktionen repräsentieren, wobei jede der virtuellen biologischen Reaktionen, die von jeder der Vorlagendaten repräsentiert werden, jedem der Sätze von Parameterwerten entspricht,
wobei das Erlangungsmittel eingerichtet ist, den Satz von Parameterwerten und die Vorlagendaten von dem Vorlagenspeichermittel zu lesen.

9.  Vorhersagesystem einer biologischen Reaktion nach Anspruch 6 oder 7, wobei
das Erlangungsmittel

ein Anfangsgruppenerzeugungsmittel zum Erzeugen einer Mehrzahl von Anfangsgruppen des Parameterwerts; und
ein Ausführungsmittel eines genetischen Algorithmus zum Ausführen eines genetischen Algorithmus an der Mehrzahl von Anfangsgruppen des Parameterwerts, die durch das Anfangsgruppenerzeugungsmittel erzeugt sind, und Erzeugen einer Mehrzahl von Sätzen von Parameterwerten enthält.

10. Verfahren zum Vorhersagen einer biologischen Reaktion, umfassend die folgenden Schritte:

Aufnehmen einer Eingabe aktueller Messdaten eines Subjekts;
Erzeugen eines virtuellen biologischen Organs, das virtuell in einem Computersystem unter Verwendung der eingegebenen aktuellen Messdaten konstruiert wird, wobei das virtuelle biologische Organ einer Funktion eines biologischen Organs des Subjekts entspricht,
Erlangen einer virtuellen biologischen Reaktion, die durch das virtuelle biologische Organ angezeigt wird, wenn eine Eingabe dem erzeugten virtuellen biologischen Organ zugeführt wird; und
Ausgeben der erlangten virtuellen biologischen Reaktion.

11. Computerprogrammprodukt, um es einem Computer zu ermöglichen, die biologische Reaktion vorherzusagen, umfassend:

ein computerlesbares Medium und
Softwareanweisungen auf dem computerlesbaren Medium, um es dem Computer zu ermöglichen, vorbestimmte Abläufe durchzuführen, umfassend:

Aufnehmen einer Eingabe von aktuellen Messdaten eines Subjekts;
Erzeugen eines virtuellen biologischen Organs, das virtuell in einem Computersystem unter Verwendung der eingegebenen aktuellen Messdaten konstruiert wird, wobei das virtuelle biologische Organ einer Funktion eines biologischen Organs des Subjekts entspricht,
Erlangen einer virtuellen biologischen Reaktion, die durch das virtuelle biologische Organ angezeigt wird, wenn eine Eingabe dem erzeugten virtuellen biologischen Organ zugeführt wird; und
Ausgeben der erlangten virtuellen biologischen Reaktion.

**Revendications**

1. Système de prédiction de réponse biologique comprenant:

   un moyen d'entrée (130) pour recevoir des données de mesure effective d'un sujet;
   un moyen de génération (110a) d'organe biologique virtuel pour générer un organe biologique virtuel élaboré virtuellement dans un système informatique en utilisant les données de mesure effective introduites, l'organe biologique virtuel correspondant à une fonction d'un organe biologique du sujet;
   un moyen d'acquisition (110a) de réponse biologique virtuelle pour faire l'acquisition d'une réponse biologique virtuelle indiquée par l'organe biologique virtuel lorsqu'on procure une entrée à l'organe biologique virtuel généré; et
   un moyen de sortie (110h, , 120) pour délivrer en sortie la réponse biologique virtuelle acquise.

2. Système de prédiction de réponse biologique selon la revendication 1, comprenant en plus:

   un moyen d'établissement de valeur d'entrée pour établir une valeur d'entrée à l'organe biologique virtuel; où
   le moyen d'acquisition (110a) de réponse biologique virtuelle est configuré pour faire l'acquisition de la réponse biologique virtuelle de l'organe biologique virtuel auquel la valeur d'entrée établie par le moyen d'établissement de valeur d'entrée est pourvue.

3. Système de prédiction de réponse biologique selon la revendication 1 ou 2, comprenant en plus:

   un moyen d'établissement de délai d'acquisition pour établir un délai afin de faire l'acquisition de la réponse biologique virtuelle de l'organe biologique virtuel; où
   le moyen d'acquisition (110a) de réponse biologique virtuelle est configuré pour faire l'acquisition de la réponse biologique virtuelle indiquée par l'organe biologique virtuel dans le délai établi par le moyen d'établissement de délai d'acquisition.

4. Système de prédiction de réponse biologique selon l'une quelconque des revendications 1 à 3, dans lequel l'organe biologique virtuel inclut un bloc (1, 2, 3, 4) indiquant la fonction de l'organe biologique associé au métabolisme du glucose et/ou de l'insuline; et
   le moyen d'acquisition (110a) de réponse biologique virtuelle est configuré pour calculer la réponse biologique virtuelle en utilisant l'organe biologique virtuel sur la base de la valeur d'entrée du bloc (1, 2, 3, 4).

5. Système de prédiction de réponse biologique selon l'une quelconque des revendications 1 à 4, comprenant en plus:

   une mémoire (140) pour stocker un modèle mathématique qui comprend plusieurs paramètres et représente une fonction d'un organe biologique, où
   le moyen de génération (110a) d'organe biologique virtuel est configuré pour générer plusieurs valeurs des paramètres du modèle mathématique en utilisant les données de mesure effective du sujet, le modèle mathématique auquel les valeurs des paramètres sont appliquées représentant la fonction de l'organe biologique du sujet.

6. Système de prédiction de réponse biologique selon la revendication 5, dans lequel le moyen de génération (110a) d'organe biologique virtuel inclut:

   un moyen d'acquisition pour faire l'acquisition de plusieurs ensembles de valeurs des paramètres;
   un moyen de comparaison pour comparer chaque réponse biologique virtuelle générée lorsque les nombreux ensembles de valeurs des paramètres acquises par le moyen d'acquisition sont appliqués au modèle mathématique, avec les données de mesure effective du sujet reçues par le moyen d'entrée; et
   un moyen de sélection pour sélectionner l'un des nombreux ensembles de valeurs des paramètres acquises par le moyen d'acquisition sur la base du résultat de comparaison du moyen de comparaison.

7. Système de prédiction de réponse biologique selon la revendication 6, dans lequel Le moyen de sélection est configuré pour sélectionner l'ensemble de valeurs des paramètres correspondant à la réponse biologique virtuelle ayant le taux de correspondance le plus élevé par rapport aux données de mesure effective du sujet.

**8.** Système de prédiction de réponse biologique selon la revendication 6 ou 7, comprenant en plus:

un moyen de stockage de modèles pour stocker plusieurs ensembles de valeurs des paramètres et plusieurs données modèles représentant les réponses biologiques virtuelles, où chacune des réponses biologiques virtuelles représentée par chacune des données modèles correspond à chacun des ensembles de valeurs des paramètres,

où le moyen d'acquisition est configuré pour lire l'ensemble de valeurs des paramètres et les données modèles à partir du moyen de stockage de modèles.

**9.** Système de prédiction de réponse biologique selon la revendication 6 ou 7, dans lequel
le moyen d'acquisition inclut,
un moyen de génération de groupes initiaux pour générer plusieurs groupes initiaux de la valeur des paramètres; et
un moyen d'exécution d'algorithme génétique pour exécuter un algorithme génétique sur les nombreux groupes initiaux de la valeur des paramètres générés par le moyen de génération de groupes initiaux, et générer plusieurs ensembles de valeurs des paramètres.

**10.** Procédé pour prédire une réponse biologique comprenant les étapes qui consistent à:

recevoir une entrée de données de mesure effective d'un sujet;
générer un organe biologique virtuel élaboré virtuellement dans un système informatique en utilisant les données de mesure effective introduites, l'organe biologique virtuel correspondant à une fonction d'un organe biologique du sujet
faire l'acquisition d'une réponse biologique virtuelle indiquée par l'organe biologique virtuel lorsqu'on procure une entrée à l'organe biologique virtuel généré; et
délivrer en sortie la réponse biologique virtuelle acquise.

**11.** Produit de programme informatique pour permettre à un ordinateur de prédire la réponse biologique comprenant:

un support lisible par ordinateur, et
des instructions logicielles, sur le support lisible par ordinateur, pour permettre à l'ordinateur d'effectuer des opérations prédéterminées comprenant le fait de:

recevoir une entrée de données de mesure effective d'un sujet;
générer un organe biologique virtuel élaboré virtuellement dans un système informatique en utilisant les données de mesure effective introduites, l'organe biologique virtuel correspondant à une fonction d'un organe biologique du sujet
faire l'acquisition d'une réponse biologique virtuelle indiquée par l'organe biologique virtuel lorsqu'on procure une entrée à l'organe biologique virtuel généré; et
délivrer en sortie la réponse biologique virtuelle acquise.

**FIG.1**

EP 1 859 731 B1

**FIG.2**

**FIG.3**

# FIG.4

Glucose absorption RG — 5

6
Blood glucose level BG

8
Net glucose release from liver SGO

Insulin secretion rate SR — 7

Post liver insulin SRpost

9

EP 1 859 731 B1

FIG.5

FIG.6

**FIG.7**

```
                    ┌─────────────────┐
                    │      START      │
                    └────────┬────────┘
                             │                    S1
                             ▼
              ┌──────────────────────────────┐
              │  Generate biological model   │
              │   based on OGTT test result  │
              └──────────────┬───────────────┘
                             │                    S2
                             ▼
              ┌──────────────────────────────┐
              │  Input conditions different from │
              │  OGTT test to biological model   │
              └──────────────┬───────────────┘
                             │                    S3
                             ▼
              ┌──────────────────────────────┐
              │  Output prediction values of │
              │  blood glucose level and blood│
              │     insulin concentration    │
              └──────────────┬───────────────┘
                             │
                             ▼
                    ┌─────────────────┐
                    │       END       │
                    └─────────────────┘
```

# FIG.8

Input time-series data of OGTT glucose and OGTT insulin — S1-1

Generate template and register in database

Compare each time-series data of template database and OGTT time-series data, and extract template having minimum error summation used in similarity determination — S1-2

Time-series data

Template database — DB

Acquire parameter set corresponding to template time-series data having high similarity from template database — S1-3

END

FIG.9A

FIG.9B

Template database DB

| Template | Parameter set |
|----------|---------------|
| T1 | PS#01 |
| T2 | PS#02 |
| T3 | PS#03 |
| ⋮ | ⋮ |

# FIG.10

Blood glucose level (template)    [mg/dl]

FIG.11A

Blood insulin concentration (template)    [(u/ml]

FIG.11B

Error (blood glucose level) [mg/dl]

FIG.12A

FIG.12B

Glucose absorption rate [mg/kg/min]

FIG.13A

**FIG.13B**

Blood glucose level [mg/dl]

FIG.14A

Blood insulin concentration    [(U/ml]

FIG.14B

Blood glucose level　[mg/dl]

FIG.15A

FIG.15B

# FIG.16

```
        ┌─────────────────────┐
        │        START        │
        └─────────────────────┘
                   │
                   ▼                          S10
   ┌──────────────────────────────┐
   │  Generate biological model   │
   │  based on OGTT test result   │
   └──────────────────────────────┘
                   │
                   ▼                          S11
   ┌──────────────────────────────┐
   │   Set calculation time of    │
   │   output of biological model │
   └──────────────────────────────┘
                   │
                   ▼                          S12
   ┌──────────────────────────────┐
   │  Output prediction values    │
   │  of blood glucose level and  │
   │         blood insulin        │
   └──────────────────────────────┘
                   │
                   ▼
        ┌─────────────────────┐
        │         END         │
        └─────────────────────┘
```

FIG.17A

Blood insulin concentration    [(U/ml]

FIG.17B

FIG.18A

Blood glucose level [mg/dl]

FIG.18B

FIG.19A

Blood insulin concentration [μU/ml]

**FIG.19B**

Blood glucose level   [mg/dl]

FIG.20A

Blood insulin concentration   [μU/ml]

FIG.20B

FIG.21A

Blood insulin concentration [µU/ml]

FIG.21B

**EP 1 859 731 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005328924 A

- WO 9728737 A